# EUROPEAN PATENT APPLICATION

(11) **EP 2 199 293 A1**
(43) Date of publication of application: **23.06.2010**
(21) Application number: 08382084.5
(22) Date of filing: 22.12.2008
(51) Int. Cl.: C07D 471/04, A61K 31/517, A61P 25/00

(54) **One-step process for preparing paliperidone and its oxalate salt**

(71) Applicant: Chemo Ibérica, S.A., 08028 Barcelona (ES)
(72) Inventor: Castaldi, Graziano, 28072, Briona (NO) (IT); Rasparini, Marcello, 27010, Cura Carpignano (PV) (IT); Marras, Giovanni, 28100, Novara (Novara) (IT); Banfi, Luca, 16148, GENOVA (IT); De Moliner, Fabio, 16014, CAMPOMORONE (Ge) (IT); Musumeci, Francesca Michella, 16032, CAMOGLI (GE) (IT); Riva, Renata, 16148, GENOVA (IT)
(74) Representative: ZBM Patents

(57) **Abstract**

The present invention relates to a one-step process for the preparation of the antipsychotic agent Paliperidone by oxidation of Risperidone, and to Paliperidone oxalate or a hydrate, solvate or polymorph thereof.

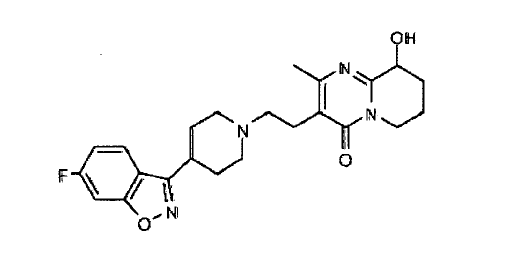

## Description

### Field of the invention

The present invention relates to a one-step process for the preparation of the antipsychotic agent Paliperidone by oxidation of Risperidone, and to Paliperidone oxalate or a hydrate, solvate or polymorph thereof.

### Background of the invention

Paliperidone is a potent antagonist of neurotransmitters serotonin and dopamine. Antagonizing said mediators will suppress or relieve a variety of symptoms associated with phenomena induced by the excessive release of these mediators.

Serotonin antagonists are reportedly effective in combating psychoses, aggressive behaviour, anxiety, depression and migraine. Central acting serotonin antagonists appear to improve the negative symptoms of schizophrenia, e.g. anergy, apathy, social withdrawal and depressive mood, and also to reduce the incidence of extrapyramidal side-effects (EPS) during maintenance therapy with classical neuroleptics, i.e. dopamine antagonists.

Dopamine receptor antagonists are known to have neuroleptic properties, for example, they counteract the positive symptoms of schizophrenia, e.g. hallucinations, delusional thinking, severe excitement and unusual behaviour.

Combined serotonin-dopamine antagonists are especially interesting as they appear to offer relief of both the positive and negative symptoms of schizophrenia, with low EPS liability. Actually, these drugs display a decreased propensity to cause extrapyramidal side effects and an absence of sustained prolactin elevation.

Therapeutic indications for using Paliperidone therefore are mainly in the CNS area, particularly as potent antipsychotic agent and especially as agents useful in treating chronic psychoses, schizophrenia and bipolar disorders. Atypical antipsychotics, second-generation drugs, like Paliperidone, are now considered to be first line treatments for schizophrenia and are gradually replacing the typical antipsychotics.

Paliperidone also appear to be a useful therapeutic agent for combating autism. Therapeutic applications in the gastrointestinal field comprise its use as, for instance, anti-diarrhoeals, inhibitors of gastro-oesophageal reflux and particularly antiemetics, e.g. in cancer patients receiving chemotherapy and radiation treatment. Further, serotonin is a potent broncho- and vasoconstrictor and thus the present antagonist may be used against hypertension and vascular disorders. In addition, serotonin antagonists have been associated with a number of other properties such as, the suppression of appetite and promotion of weight loss, which may prove effective in combating obesity; and also the alleviation of withdrawal symptoms in addicts trying to discontinue drinking and smoking habits.

Chemically, Paliperidone is the primary active metabolite of the older atypical antipsychotic Risperidone. Risperidone is extensively metabolized in the liver: the main metabolic pathway is through hydroxylation into 9-hydroxy Risperidone (Paliperidone), by the enzyme Cytochrome P450 2D6 (CYP 2D6). Paliperidone has a pharmacological profile and potency comparable with that of the parent drug Risperidone, but has a longer elimination half-life. Risperidone is distributed to and eliminated from the brain tissues more rapidly than its metabolite Paliperidone.

Paliperidone is a compound of formula (I) 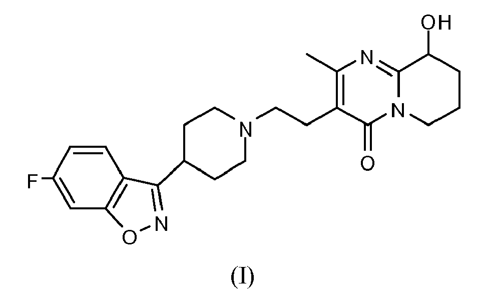 chemically known as 3-[2-[4-(6-fluoro-1,2-benzisoxazol-3-yl)-1-piperidinyl]ethyl]-6,7,8,9-tetrahydro-9-hydroxy-2-methyl-4*H*-pyrido[1,2-a]pyrimidin-4-one, which is disclosed in EP 368 388 B1 and marketed worldwide under the trade name Invega^{®}.

Very few methods have been disclosed till now for the preparation of Paliperidone.

The process disclosed in the patent EP 368 388 involves the condensation of 2-amino-3-hydroxypyridine with 3-acetyldihydrofuran-2(3*H*)-one in presence of phosphorous oxychloride to provide 3-(2-chloroethyl)-2-methyl-9-hydroxy-4*H*-pyrido[1,2-a]pyrimidin-4-one. Hydrogenation of the pyridine ring and subsequent alkylation reaction with 6-fluoro-3-piperidino-1,2-benzisoxazole afford the target compound.

The patent application WO 2008/024415 involves a nucleophilic substitution of the final hydroxyl group in 3-(2-hydroxyethyl)-2-methyl-9-benzyloxy-4*H*-pyrido[1,2-a]pyrimidin-4-one in presence of phosphorous oxychloride. Subsequent deprotection of the benzyl group and hydrogenation of the pyridine ring provide -(2-chloroethyl)-2-methyl-9-hydroxy-4*H-*pyrido[1,2-a]pyrimidin-4-one. This compound is then submitted to alkylation with 6-fluoro-3-piperidino-1,2-benzisoxazole, to give Paliperidone.

International patent application WO 2008/144073 provides a process for the preparation of paliperidone comprising enzymatic hydroxylation of risperidone with at least one oxidoreductase enzyme.

Paliperidone prepared by the processes known up till now can only be obtained in a satisfactory quality after running through a number of process steps or using enzymes which include high cost and need of enzyme isolation and purification. There is therefore the need for an alternative synthesis for industrial application, which makes use also of safer reagents.

An object of the present invention is to provide an economical one-step process for the manufacture of Paliperidone that avoids the above-identified problems.

### Summary of the invention

It has now been found a process for the preparation of Paliperidone in a straightforward manner.

The method of the present invention provides a one-step process for preparing Paliperidone of formula (I), or a pharmaceutically acceptable salt, a hydrate, a solvate, a polymorph thereof, by oxidising Risperidone of formula (II), or a pharmaceutically acceptable salt, a hydrate, a solvate, a polymorph thereof 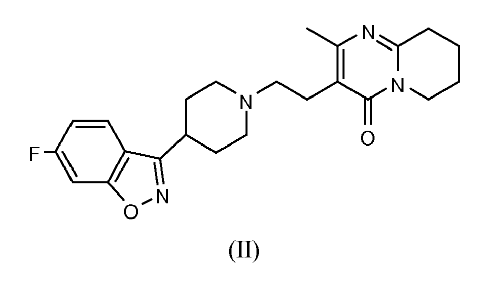 with an oxidising agent, in the presence of a basic agent and optionally in the presence of an agent that it is able to reduce the formed hydroperoxide to the desired alcohol.

In another embodiment, the present invention provides the use of Risperidone, or a pharmaceutically acceptable salt, a hydrate, a solvate, a polymorph thereof, as intermediate in the preparation of Paliperidone.

In another embodiment, the invention provides a process for purifying Paliperidone via oxalate salt formation.

In another embodiment, the present invention provides Paliperidone oxalate or a hydrate, solvate or polymorph thereof.

In another embodiment, the invention provides a pharmaceutical composition comprising Paliperidone obtained with the process according to the present invention optionally together with at least one pharmaceutically acceptable excipient.

In another embodiment, the present invention provides the use of Paliperidone obtained with the process according to the present invention, for the preparation of a medicament.

### Description of figures

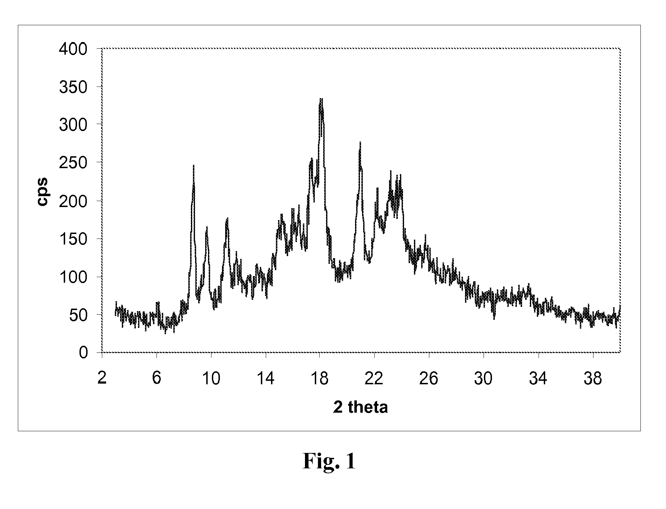

Fig. 1 provides an XRPD pattern of Paliperidone oxalate in amorphous form.

### Detailed description of the invention

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

The term "alkyl" refers to a straight or branched hydrocarbon having from 1 to 6 carbon atoms. Examples of alkyl groups include, without limitation, methyl, ethyl, *n*-propyl, isopropyl, *n*-butyl, *sec*-butyl, isobutyl, *tert*-butyl, *n*-pentyl, *n*-hexyl, and the like.

The term "pharmaceutically acceptable salt(s)", as used herein, unless otherwise indicated, includes salts of basic groups which may be present in the compounds of the present invention. The compounds prepared according to the present invention that are basic in nature are capable of forming a wide variety of salts with various inorganic and organic acids. The acids that may be used to prepare pharmaceutically acceptable acid addition salts of such basic compounds are those that form non-toxic acid addition salts i.e., salts containing pharmacologically acceptable anions, such as the hydrochloride, hydrobromide, hydroiodide, nitrate, sulfate, bisulfate, phosphate, acid phosphate, isonicotinate, acetate, lactate, salicylate, citrate, acid citrate, oxalate, tartrate, pantothenate, bitartrate, ascorbate, succinate, maleate, gentisinate, fumarate, gluconate, glucuronate, saccharate, formate, benzoate, glutamate, methanesulfonate, ethanesulfonate, benzenesulfonate, p-toluenesulfonate andpamoate [i.e., 1,1'-methylene-bis-(2-hydroxy-3-naphthoate)] salts. The compounds prepared according to the present invention may form pharmaceutically acceptable salts with various amino acids, in addition to the acids mentioned above.

Salts may be prepared according to methods well known in the art. One may prepare a pharmaceutically acceptable base salt by contacting Paliperidone with a sufficient amount of a desired acid to produce a nontoxic salt. If the salt precipitates from solution, it may be isolated by filtration; otherwise, the salt may be recovered by evaporating the solvent.

The term "solvate" refers to a molecular complex comprising a disclosed or claimed compound and a stoichiometric or non-stoichiometric amount of one or more solvent molecules (*e.g*., ethanol).

The term "hydrate" refers to a solvate comprising a disclosed or claimed compound and a stoichiometric or non-stoichiometric amount of water.

The term "polymorph" refers to substances that have the same chemical formula, but different crystal structures.

It is contemplated that the compounds of the present method can be found or isolated in the form of hydrates or solvates, in different polymorphic forms, i.e., different crystalline forms, which are considered to fall within the scope of the present invention.

Generally, the chemical transformations described throughout the specification may be carried out using substantially stoichiometric amounts of reactants, though certain reactions may benefit from using an excess of one or more of the reactants. Additionally, many of the reactions disclosed throughout the specification, may be carried out at room temperature, but particular reactions may require the use of higher or lower temperatures, depending on reaction kinetics, yields, and the like. Furthermore, many of the chemical transformations may employ one or more compatible solvents, which may influence the reaction rate and yield. Depending on the nature of the reactants, the one or more solvents may be polar protic solvents, polar aprotic solvents, non-polar solvents, or some combination.

The process of the present invention is illustrated in the following reaction Scheme 1, wherein Risperidone (II) is converted into Paliperidone (I), by reaction with an oxidising agent, such as oxygen, air, N-sulfonyl aziridines (Davis aziridines), a molybdenum peroxide derivative, such as MoO₅.Py.HMPA (MoOPH), in a suitable solvent. Preferably, the oxidising agent is air. The method of the present invention makes use of generally inexpensive and readily available reagents.

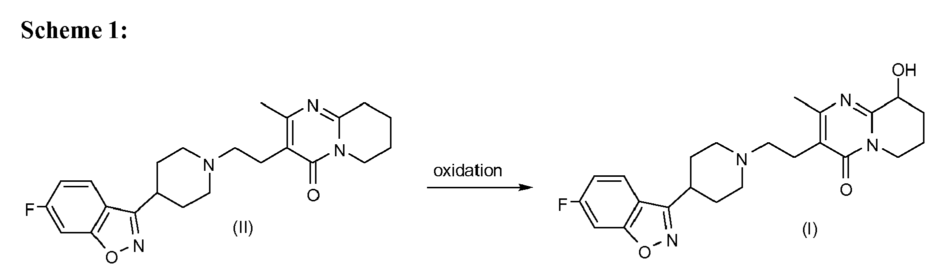

Suitable solvents for the oxidation reaction are amide solvents, such as dimethylformamide, dimethylacetamide; ethers, such as tetrahydrofuran, dimethoxyethane; halogenated solvents, such as dichloromethane, 1,2-dichloroethane, chloroform. Preferably, the solvents for the oxidation reaction are tetrahydrofuran or dichloromethane, more preferably dichloromethane.

The reaction is carried out in presence of a basic agent, at a suitable temperature. Examples of basic agent are strong bases, such as metal hydrides, for instance sodium, potassium or lithium hydride; silicon-based amides, such as sodium or lithium bis(trimethylsilyl)amide; lithium diisopropylamide; or magnesium-based amides, such as diisopropylamide magnesium bromide. A preferred base is lithium bis(trimethylsilyl)amide. The basic agent can be employed in a molar ratio to the compound (II) comprised between 1.0 and 2.0, preferably between 1.0 and 1.5. The temperature of the oxidation reaction may range from room temperature to -78°C, preferably the temperature is between 0°C and -78°C, more preferably between -40°C and -78°C.

The oxidation of Risperidone (II) to Paliperidone (I) can be optionally carried out in the presence of an agent that it is able to reduce the formed hydroperoxide to the desired alcohol, for instance a trialkyl phosphite, such as triethyl or trimethyl phosphite, preferably trimethyl phosphite; phosphines, such as trimethylphosphine, tributylphosphine, triphenylphosphine; or dimethylsulfide.

Paliperidone can be effectively separated from the reaction mixture and purified by employing a conventional method well known to those skilled in the art, such as extraction, recrystallization, column chromatography, or by transforming then into a salt or by washing with an organic solvent or with an aqueous solution, eventually adjusting pH. Preferably, Paliperidone can be purified *via* salt formation, according to methods well known in the art. More preferably, Paliperidone is purified *via* oxalate salt formation in a suitable solvent, for instance a polar protic solvent, such as ethanol, methanol, preferably methanol. The isolated solid, can be recovered with methods well known to those skilled in the art for the separation of the solid from the mother liquor, for example by filtration, with or without the assistance of pressure and/or vacuum, or by centrifugation, or by decantation. The collected solids are washed with at least a suitable solvent and dried by conventional methods well known to those skilled in the art. Paliperidone oxalate is a new compound and is also object of the present invention. Preferably, Paliperidone oxalate is in an amorphous form, characterised by the X-ray powder diffraction pattern shown in Figure 1.

Paliperidone obtained with the method of the present invention can be converted according to methods well known in the art into a pharmaceutically acceptable salt or converting the salt thereof into the free Paliperidone. One may regenerate the free base by contacting the acid addition salt with a base. Though certain physical properties of the free base and its respective acid addition salt may differ (*e.g*., solubility, crystal structure, hygroscopicity, etc.), a compound's free base and acid addition salt are otherwise the same for purposes of this disclosure.

Paliperidone, obtained with the process according to the present invention, may be used for the preparation of a medicament.

The starting material Risperidone (I) is a commercial compound or may be prepared according to methods well known in the art. For instance, it can be prepared as described in the patent EP 196 132, wherein is obtained by condensation between 3-(2-chloroethyl)-6,7,8,9-tetrahydro-2-methyl-4*H*-pyrido[1,2-a]pyrimidin-4-one and 6-fluoro-3-(4-piperidinyl)-1,2-benzisoxazole.

The present invention provides the use of Risperidone, or a pharmaceutically acceptable salt, a hydrate, a solvate, a polymorph thereof, as intermediate in the preparation of Paliperidone.

While the present invention has been described in terms of its specific embodiments, certain modifications and equivalents will be apparent to those skilled in the art and are included within the scope of the present invention.

In the following, the present invention shall be illustrated by means of some examples, which are not construed to be viewed as limiting the scope of the invention.

### Examples

The following abbreviations refer respectively to the definitions below:
LiHMDS (Lithium bis(trimethylsilyl)amide).

XRPD spectra were performed by means of an APD 2000 Ital Structures diffractometer at 25°C, using a CuKα tube (40 kV, 30 mA, λ = 1.5406 Å) as the X-ray source. Data collection was made in 2θ step scan mode and in Bragg-Brentano configuration, at a scan speed of 0.02°/s in the range of 3° to 40° in 2θ. The samples were placed on an aluminium sampler.

### Example 1

### Preparation of LiHMDS

In a 25 mL round bottomed flask few crystals of 2,2'-bipyridyl are placed and the system is placed under an argon atmosphere. Anhydrous toluene (2 mL) is added, the solution is cooled to -10 °C and freshly distilled hexamethyldisilazane (735 µL, 3.52 mmol) is added, followed by *n*-BuLi (1.6 M solution in hexane, 2.0 mL, 3.2 mmol). The mixture is stirred at -10 °C for 30 minutes, to obtain a *ca*. 0.68 M red solution ofLiHMDS.

### Example 2

### Oxidation reaction

A 50 mL two-necked round bottomed flask is connected to a compressed air cylinder (GC grade, H₂O < 5 ppm) *via* an empty Drechsel bottle. Risperidone (500 mg, 1.22 mmol) is charged under an argon atmosphere. Dry dichloromethane (20 mL) is added and the solution is cooled to -78 °C. Trimethyl phosphite (97%, 197 µL, 1.58 mmol) and LiHMDS (2.69 ml, 1.83 mmol) were added. After 5 minutes stirring, air is slowly bubbled through the solution (about 1 bubble per second) for 6-7 hours keeping the temperature at -78 °C. The reaction is monitored by TLC (elution with dichloromethane/methanol/ammonia 95:5:1 v/v, UV visualization and ninhydrin). The reaction is stopped by quenching with NH₄Cl (saturated solution in water, 5 mL) and K₂CO₃ (saturated solution in water, 5 ml). The product is extracted in dichloromethane from pH 10-11. The organic phase is washed with brine, dried over Na₂SO₄ and the solvent is removed under reduced pressure. The residue is purified by flash chromatography on silica gel (20 g) with gradient elution dichloromethane/methanol (90:10 to 86:14) obtaining the ketone by-product (43 mg, 8%) in the forerunning fractions as a yellow oil, followed by Paliperidone (342 mg, 66%) as a white foam. The product was further purified by crystallization from ethyl acetate/hexanes, providing a product with m.p. 162.2-162.4 °C.

¹H NMR (300 MHz, CDCl₃, 298K) δ 1.77 (1H, centre ofm); 1.89-2.04 (1H, m); 2.07-2.21 (6H, m); 2.25-2.42 (2H, m); 2.36 (3H, s); 2.55 and 2.79 (4H, A and X part of an AA'XX' system); 3.10 (1H, centre of m); 3.18 (2H, broad d, J 11.4 Hz); 3.87-4-03 (2H, m); 4..12 (1H, broad s); 4.51 (1H, dd, J 6.3 and 10.2 Hz); 7.07 (1H, dt, J 2.1 and 15.3 Hz); 7.25 (1H, dd, J 2.4 and 9.0 Hz); 7.72 (1H, dd, J 5.4 and 9.0 Hz).

¹³C NMR (75.4 MHz, CDCl₃, 298K) δ 18.5; 21.2; 23.9; 27.0; 34.6; 42.4; 53.4; 56.6; 67.1; 97.5 (d, ²J_{C-F} 27.0 Hz), 112.4 (d, ²J_{C-F} 25.3 Hz), 117.4; 120.5; 122.7 (d, ³J_{C-F} 11.5 Hz); 157.5; 157.9; 161.1; 162.1; 163.8; 164.0 (d, ¹J_{C-F} 248.5 Hz); 163.9 (d, ³J_{C-F} 13.2 Hz)

### Example 3

### Purification of Paliperidone via salt formation

Crude Paliperidone from the oxidation reaction of 10 g of Risperidone (theoretical yield 24.4 mmol), after aqueous work-up as described above, was dissolved in methanol (100 mL) and added of oxalic acid (1.3 g, 14.4 mmol) dissolved in methanol (10 mL).

The crystalline material was filtered and washed with methanol, obtaining Paliperidone oxalate as a colourless solid (7.2 g, 60% yield), m.p. 135-140°C, showing the XRPD pattern reported in Fig. 1.

Paliperidone was obtained in quantitative yield from the oxalate salt (7.2 g, 13.9 mmol) by treatment with 20% ammonium hydroxide and extraction in dichloromethane (50 mL), washing with brine, drying the organic extract over sodium sulphate and concentrating to a solid residue. Paliperidone was optionally recrystallized from acetonitrile.

## Claims

1. A process for preparing Paliperidone of formula (I), or a pharmaceutically acceptable salt, hydrate, solvate or polymorph thereof, 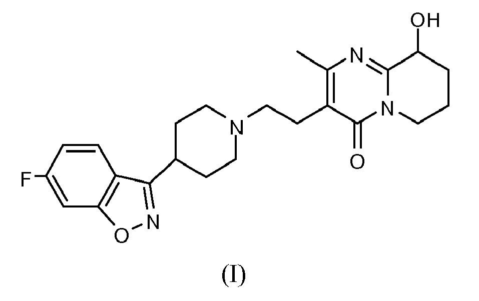 which comprises:
oxidising Risperidone of formula (II), or a pharmaceutically acceptable salt, a hydrate, a solvate, a polymorph thereof, 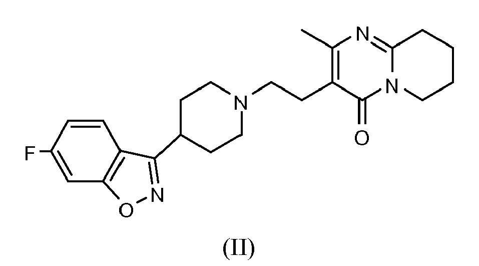 with an oxidising agent, in the presence of a basic agent and optionally in the presence of an agent that it is able to reduce the formed hydroperoxide to the desired alcohol.

2. The process according to claim 1, wherein the oxidising agent is oxygen, air, N-sulfonyl aziridines or a molybdenum peroxide derivative.

3. The process according to claim 2, wherein the oxidising agent is air.

4. The process according to claim 1, wherein the basic agent is a strong base, such as metal hydrides, silicon-based amides or magnesium-based amides.

5. The process according to claim 4, wherein the basic agent is lithium bis(trimethylsilyl)amide.

6. The process according to claim 1, wherein the oxidation is conducted in ethers, amide orhalogenated solvents.

7. The process according to claim 1, wherein the agent that it is able to reduce the formed hydroperoxide to the desired alcohol is trialkyl phosphite, phosphines or dimethylsulfide.

8. The process according to claim 7, wherein the trialkyl phosphite is triethyl or trimethyl phosphite.

9. The process according to claim 8, wherein the trialkyl phosphite is trimethyl phosphite.

10. The process according to claim 1, further comprising purifying the obtained paliperidone via oxalate salt formation.

11. Paliperidone oxalate or a hydrate, solvate or polymorph thereof.

12. Paliperidone oxalate according to claim 11, in an amorphous form **characterised by** the X-ray powder diffraction pattern shown in Figure 1.

13. Use of Paliperidone obtained with the process according to any one of claims 1 to 10, for the preparation of a medicament.

14. A pharmaceutical composition comprising Paliperidone obtained with the process according to any one of claims 1 to 10 optionally together with at least one pharmaceutically acceptable excipient.
